# EUROPEAN PATENT SPECIFICATION

(11) **EP 3 659 150 B1**
(45) Date of publication and mention of the grant of the patent: **06.03.2024**
(21) Application number: 18749323.4
(22) Date of filing: 25.07.2018
(51) Int. Cl.: G16H 30/40, G16H 30/20, G16H 40/20

(54) **DEVICE, SYSTEM, AND METHOD FOR OPTIMIZING IMAGE ACQUISITION WORKFLOWS**
VORRICHTUNG, SYSTEM UND VERFAHREN ZUR OPTIMIERUNG VON BILDERFASSUNGSARBEITSABLÄUFEN
DISPOSITIF, SYSTÈME ET PROCÉDÉ POUR OPTIMISER LE FLUX DE PRODUCTION D'ACQUISITION D'IMAGES

(30) Priority: 25.07.2017 US 201762536620 P
(43) Date of publication of application: 03.06.2020
(73) Proprietor: Koninklijke Philips N.V., 5656 AG Eindhoven (NL); The University of Chicago, Chicago, IL 60637 (US)
(72) Inventor: SEVENSTER, Merlijn, 5656 AE Eindhoven (NL); SCHADEWALDT, Nicole, 5656 AE Eindhoven (NL); TAHMASEBI, Amir, Mohammad, 5656 AE Eindhoven (NL); CHANG, Paul, Joseph, 5656 AE Eindhoven (NL)
(74) Representative: Philips Intellectual Property & Standards
(86) International application number: PCT/EP2018/070231
(87) International publication number: WO 2019/020717

(56) References cited:
- US-A1- 2009 006 131
- US-A1- 2016 350 919

## Description

### Background Information

A physician may provide healthcare services to patients using a variety of different procedures and recommending a variety of different tests to be performed in pursuit of a proper treatment. For example, the physician may recommend that a further examination of a target area on the body of a patient is needed. Accordingly, the physician may recommend that the patient schedule an image acquisition procedure such as a computed tomography (CT) scan, a magnetic resonance (MR) scan, etc. As the imaging acquisition procedure is performed by another party (such as an image interpreter and not the physician), the treatment process waits for the results of the imaging acquisition procedure to reach the physician for a subsequent determination to be made. The physician may be a referring physician who provides an image order to the image interpreter who performs the proper operations in the image acquisition procedure.

In the course of performing the image acquisition procedure, the image interpreter may capture images of a desired area of the patient using a variety of different procedures and with a variety of different approaches. As those skilled in the art will understand, the image acquisition procedure may comprise a plurality of sequences where each sequence interrogates the anatomy of the desired area under different settings. For example, the settings may include contrast use, slice thickness, pulse sequences (for MR scans), etc. The settings may also be used in different combinations of modality parameters such as repetition time, inversion time, flip angle, etc. Accordingly, there may be a large number of different sequences that may be used where a specific sequence has a unique set of characteristics in capturing an image.

To manage an appropriate acquisition, select sequences may be bundled into a protocol or an exam card (hereinafter collectively referred to as a "protocol"). The protocol may be selected by the image interpreter based on the image order received from the referring physician where the image order may include the expectations and needs that are to be included in the results of the image acquisition procedure. The image order may therefore be used by the image interpreter to determine one or more protocols to be used where each protocol includes one or more sequences. Specifically, each protocol may have associated indicators that specify the conditions in which the protocol is to be used for an image acquisition procedure.

The protocols that are selected for the image acquisition procedure may be specific to the image interpreter, to the institute in which the image acquisition procedure is performed, to a region, etc. The protocols may also evolve over time. If properly managed, new protocols may be added while outdated protocols may be removed. For example, the set of available protocols may change based on new guidelines, novel publications, recent research, etc. Furthermore, if properly managed, each sequence in each protocol may also be modified in a similar manner. For example, a protocol may include a plurality of sequences but may be modified to alter an existing sequence, add a new sequence, remove an outdated sequence, etc. Accordingly, the protocols that are being used potentially become cluttered, particularly when more and more sequences are added to the protocol to remain contemporary with modern practice.

In view of the one or more protocols that are used and the one or more sequences in each of the protocols, the acquisition of images using the sequences may consume significant time. This time affects the patient, the technologist, and the image interpreter as well as occupying the modality or equipment being used. Therefore, without properly managing the protocols, the patient may become unnecessarily exposed to further contrast and unnecessary image capture. The additional sequences may encumber the image interpreter during interpretation. In fact, there is an estimated 5 to 10% of the exams performed for image orders that include an incorrect protocol (and the associated sequences). When incorrect protocols are used, the images captured with the corresponding sequences may never even be used or read by the image interpreter. This leads to an unnecessary overhead in the time used for an image acquisition procedure with no value being added for use of this time. US 2009/006131 A1 discloses a method and system for optimizing workflows and determining future imaging procedures. However, it merely aims to provide an future imaging procedure based on an analyses of the EMR and other parameters, but not on the actual usage of past images.

US 2016/350919 A1 involves obtaining image data from imaging device and non-image data that are associated with a medical imaging study. A deep learning model is selected to apply automated image recognition of a particular medical condition on the image data. The characteristics of the particular medical condition are recognized from image content of the image data. An electronic workflow is modified for performing a diagnostic evaluation of the medical imaging study. The electronic workflow is modified based on the recognized characteristics of the particular medical condition,

Whether or not improper protocols are being used, there is also an even larger estimate of exams that potentially benefit from reducing the number of sequences in the protocols. Without proper management of the sequences in the protocols, irrelevant images captured with unnecessary sequences further clutter the results of the image acquisition procedure which ultimately decreases efficiency and throughput. With an abundance of irrelevant images, the image interpreter may be hindered from performing the interpretation. In fact, inexperienced personnel may lose further time in reviewing these irrelevant images.

It is therefore mandatory that only those images acquired with the appropriate sequences that serve a medical purpose are acquired in the image acquisition procedure. The determination of relevance and medical purpose is a complex decision that depends on a variety of different factors (e.g., the patient's clinical history, the reason for exam, the clinical question, recent lab results, previously consulted sequence, etc.).

### Summary

The invention is defined by the appended claims.

The exemplary embodiments are directed to a method, comprising: at a workflow server: receiving data associated with previous image acquisition procedures that have been performed, the image acquisition procedures having respective image orders, the image orders having respective contexts, each of the image acquisition procedures having used at least one sequence defining settings to capture an image; determining at least one metric associated with a use of a first one of the at least one sequence for a first one of the contexts; and generating a rule indicative of whether the first sequence is to be included or excluded for a subsequent image acquisition procedure having the first context based on the at least one metric.

The exemplary embodiments are directed to a workflow server, comprising: a transceiver communicating via a communications network, the transceiver configured to receive data associated with previous image acquisition procedures that have been performed, the image acquisition procedures having respective image orders, the image orders having respective contexts, each of the image acquisition procedures having used at least one sequence defining settings to capture an image; a memory storing an executable program; and a processor that executes the executable program that causes the processor to perform operations, comprising: determining at least one metric associated with a use of a first one of the at least one sequence for a first one of the contexts; and generating a rule indicative of whether the first sequence is to be selected for a subsequent image acquisition procedure having the first context based on the at least one metric.

The exemplary embodiments are directed to a method, comprising: at a workflow server: receiving an image order from a referring physician to have an image acquisition procedure performed on a patient, the image order having an associated context; determining whether the associated context corresponds to a previous context of previous image acquisition procedures that have been performed; when the associated context corresponds to the previous context, determining at least one rule associated with the previous context, the at least one rule indicating whether a respective sequence is to be included or excluded for the image acquisition procedure, each of the at least one rule being based on at least one metric associated with a use of the respective sequence for the previous context; and determining how to incorporate the respective sequence in the image acquisition procedure based on the at least one rule.

### Brief Description of the Drawings

Fig. 1 shows a system according to the exemplary embodiments.
Fig. 2 shows a workflow server of Fig. 1 according to the exemplary embodiments.
Fig. 3 shows a method for managing protocols and sequences of the protocols according to the exemplary embodiments.
Fig. 4 shows a method for selecting a protocol and/or a sequence for an image order according to the exemplary embodiments.

### Detailed Description

The exemplary embodiments may be further understood with reference to the following description and the related appended drawings, wherein like elements are provided with the same reference numerals. The exemplary embodiments are related to a device, a system, and a method for optimizing a workflow for an image acquisition procedure. Specifically, the exemplary embodiments are configured to automatically determine or recommend to an image interpreter one or more protocols and/or one or more sequences that are to be used in response to an image order from a referring physician. Based on the characteristics of the image order and a selection of the protocols/sequences using a rules based approach, the exemplary embodiments may be configured to automatically determine or generate a recommendation on how an image acquisition procedure is to proceed.

It is noted that the exemplary embodiments are described with respect to optimizing the workflow for an image acquisition procedure and an associated reading environment for an image interpreter to perform the various sequences in generating results for an image order for a referring physician. However, the image acquisition procedure, the sequences, associated protocols including the sequences, and the image order are only exemplary. The exemplary embodiments may be modified to be used with any process flow in which a referring physician has an order for another technician to generate results using a procedure that selects one or more different operations with corresponding settings.

The exemplary embodiments provide a mechanism that uses historical data of image acquisition procedures to update and maintain a manner in which subsequent image acquisition procedures are to be performed in an efficient manner by using operations that generate little to no irrelevant results or outputs. The mechanism according to the exemplary embodiments may track acquired images from previous image acquisition procedures and draw conclusions to identify sequences and/or protocols that are still being used or in effect which may be defined as waste operations. Specifically, sequences or protocols may be considered to be omitted from use for subsequent image acquisition procedures so that these subsequent image acquisition procedures may be performed in a more efficient manner from the capturing operations (by the technician) to all other downstream operations (interpretation by the image interpreter). Thus, the exemplary embodiments may define optimization rules for protocols/sequences and enforce or enable the appropriate reduction in the image acquisition workflow. Through this feature of the exemplary embodiments, the overhead and waste may be reduced and efficiency may be optimized. Moreover, the exemplary embodiments may be used to generate insights into the usefulness of specific sequences/protocols and provide transparency and standardization among different institutions or within the medical field.

As will be described in detail below, the exemplary embodiments may be utilized in a variety of different manners. In a first example, the exemplary embodiments may be used in an overarching manner where the optimization rules may be for an overall use of predefined protocols/sequences used for image orders matching a particular set of parameters. Accordingly, a particular sequence for a given protocol may be removed for all uses of the protocol. In a second example, the exemplary embodiments may be used in a dynamic manner where the optimization rules may filter available protocols/sequences to match a specific image order that was received for a particular patient. Accordingly, a particular selected protocol may be specified particularly for the specific image order. By eliminating particular protocols/sequences based on a semantically normalized machine-interpretable understanding of the context of the image order, a direct set up of the modality for the image acquisition procedure may be performed based on the features of the exemplary embodiments.

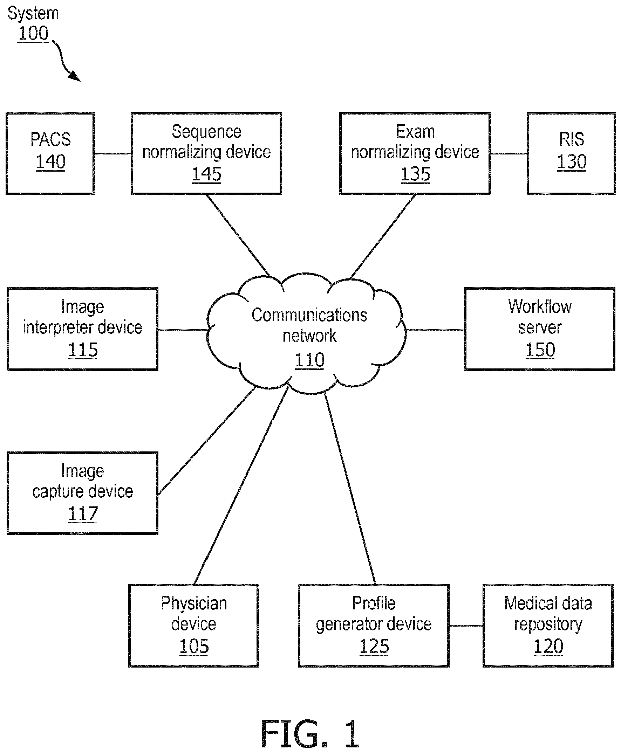

Fig. 1 shows a system 100 according to the exemplary embodiments. The system 100 relates to a communication between various components involved in requesting an image acquisition procedure, performing the image acquisition procedure, and generating results of the image acquisition procedure. Specifically, the system 100 may relate to a scenario when a physician refers the patient to an image interpreter for an image acquisition procedure to be performed to generate test results that are returned to the referring physician. The system 100 may include a physician device 105, a communications network 110, and an image interpreter device 115. As will be described in further detail below, the system 100 is configured to determine the protocols/sequences to be used in the image acquisition procedure based on predetermined parameters associated with the image order and the protocols/sequences. In a first aspect of the exemplary embodiments, the predetermined parameters may be determined. Accordingly, the system 100 may also include a medical data repository 120, a profile generator device 125, a Radiology Information System (RIS) 130, an exam normalizing device 135, a Picture Archiving and Communication System (PACS) 140, and a sequence normalizing device 145. The system 100 may further include a workflow server 150 that utilizes information from these components in determining the predetermined parameters. In a second aspect of the exemplary embodiments, the workflow server 150 may also be used in processing an image order based on the predetermined parameters.

The physician device 105 may represent any electronic device that is configured to perform the functionalities associated with a physician. For example, the physician device 105 may be a portable device such as a tablet, a laptop, etc. or a stationary device such as a desktop terminal. The physician device 105 may include the necessary hardware, software, and/or firmware to perform the various operations associated with medical treatment. The physician device 105 may also include the required connectivity hardware, software, and firmware (e.g., transceiver) to establish a connection with the communications network 110 to further establish a connection with the other components of the system 100.

The physician device 105 may be configured to enable the physician to perform the various operations associated with medical treatment. For example, the physician device 105 may schedule appointments for patients using a calendar application, may track treatments or procedures of a patient, etc. In another example, the physician device 105 may schedule or request an image acquisition procedure to be performed on the patient with an image capture device 117. More specifically, the physician device 105 may be used to generate the image order that is used for the image acquisition procedure. Subsequently, the patient may determine where and when to have the image acquisition procedure. The physician may enter various inputs to generate the image order. For example, the image order may include information (e.g., target area, expectation of types of information from the results, etc.) that may be used by the image interpreter or technician performing the image acquisition procedure (e.g., selecting a protocol or a sequence to be used). The physician device 105 may utilize any manner of generating the image order. For example, the physician device may use a free form text input, a standardized selection form, or a combination thereof. In a further example, the physician device 105 may receive test results associated with the image acquisition procedure and display the results to the physician.

The communications network 110 may be configured to communicatively connect the various components of the system 100 to exchange data. The communications network 110 may represent any single or plurality of networks used by the components of the system 100 to communicate with one another. For example, if the physician device 105 is used at a hospital, the communications network 110 may include a private network with which the physician device 105 may initially connect (e.g. a hospital network). The private network may connect to a network of an Internet Service Provider to connect to the Internet. Subsequently, through the Internet, a connection may be established to other electronic devices. For example, the workflow server 150 may be remote relative to the hospital but may be connected to the Internet. Thus, the physician device 105 may be communicatively connected to the workflow server 150. It should be noted that the communications network 110 and all networks that may be included therein may be any type of network. For example, the communications network 110 may be a local area network (LAN), a wide area network (WAN), a virtual LAN (VLAN), a WiFi network, a HotSpot, a cellular network (e.g., 3G, 4G, Long Term Evolution (LTE), etc.), a cloud network, a wired form of these networks, a wireless form of these networks, a combined wired/wireless form of these networks, etc.

The image interpreter device 115 may represent any electronic device that is configured to perform the functionalities associated with an image interpreter. For example, like the physician device 105, the image interpreter device 115 may be a portable device such as a tablet, a laptop, etc. or a stationary device such as a desktop terminal. The image interpreter device 115 may also include the necessary hardware, software, and/or firmware to perform the various operations associated with imaging procedures. The image interpreter device 115 may also include the required connectivity hardware, software, and firmware (e.g., transceiver) to establish a connection with the communications network 110 to further establish a connection with the other components of the system 100.

The image interpreter device 115 may be configured to enable the image interpreter to perform the various operations associated with image acquisition procedures. As those skilled in the art will understand, there are a plurality of different image acquisition procedures that may be performed using different imaging modalities. For example, the image acquisition procedures may be an X-ray procedure, a computed tomography (CT) procedure, a magnetic resonance imaging (MRI) procedure, an ultrasound procedure, a positron emission tomography (PET) scan procedure, a single-photon emission computed tomography (SPECT) scan procedure, etc. Each image acquisition procedure may utilize a reading environment upon which images may be captured for the image interpreter to interpret. Each of the images captured during the image acquisition procedure may be based on various settings defined in a sequence that may be included in a protocol. As will be described in further detail below, the image interpreter device 115 may receive an image order from the physician device 105 as well as further outputs from the workflow server 150 to determine the sequences and/or protocols to be used that define how the images are to be captured in the image acquisition procedure for the patient.

The image capture device 117 may represent the equipment used by a technician in capturing the images for the image acquisition procedure. As noted above, the image capture device 117 may be a specific type of equipment used for a corresponding one of the variety of different types of image acquisition procedures that are available. For example, the image capture device 117 may be a CT equipment, a MRI equipment, an ultrasound machine, etc. When the sequences/protocols to be used have been determined using the image interpreter device 115, the image capture device 117 may be provided this sequence/protocol information so that the technician may perform the appropriate sequences/protocols to capture the proper images as instructed by the image interpreter. Once the capturing portion of the image acquisition procedure is completed, the image interpreter device 115 may be provided the captured images for interpretation. Using at least one of the captured images, the image interpreter device 115 may be used by the image interpreter to generate test results from the image acquisition procedure which may then be transmitted to the physician device 105.

As will be described in further detail below, the physician device 105, the image interpreter device 115, and the image capture device 117 may include a further functionality in which data is transmitted to select ones of the other components of the system 100. As will become apparent below, the other components may include functionalities that receive data from the physician device 105, the image interpreter device 115, and/or the image capture device 117 as well as forward information to other components including the workflow server 150.

The medical data repository 120 may be a repository of medical data that may be queried for information pertaining to patients. For example, the medical data repository 120 may be configured with an application programming interface (API) from which components of the system 100 may request the medical data for a particular patient. The medical data repository 120 may be particularly directed to patient histories where each patient may have an electronic medical record (EMR) used to track the different procedures, treatments, visits, etc. of the patient. In a first example, the medical data repository 120 may include narrative reports received from or based on information from the physician device 105, the image interpreter device 115, etc. Specifically, the narrative reports may be reports related to radiology, surgery, pathology and progress reports, admission notes, etc. In a second example, the medical data repository 120 may include code lists. Specifically, the code lists may indicate various medically related information such as a problem list (e.g., ICD 10), a medical history and physicals (e.g., ICD 10), medications (e.g., RxNorm), lab work (e.g., LOINC), etc. The medical data repository 120 may receive information from any source or information carrier in compiling the EMR for the patients being tracked in the medical data repository 120. Accordingly, there may be a plurality of different information carriers from which the information may originate.

The profile generator device 125 may be a component that generates a semantically normalized clinical profile for a patient based on records kept for the patient. Specifically, a respective EMR for a particular patient may be normalized. As the information stored in the medical data repository 120 may be distributed over various types of information carriers, there may not be any consistency in the information. For example, each information carrier may not be complete or consistent with the other information carriers. In addition, the information may not be presented in a semantically normalized manner such as being codified in a background ontology that is shared between various information carriers. Specifically, if the medical data repository 120 stores narrative reports, there may be no standardized format in which the patient information is included. Accordingly, the profile generator device 125 may be configured to normalize the information from the information carriers.

The profile generator device 125 may be configured with a dedicated layer to extract a status of phenotypes from one or more of the information carriers. In this manner, the information from the information carriers may be normalized by determining whether a patient has a particular condition, has undergone a particular procedure, has had a particular treatment, etc. For example, the EMR in the medical data repository 120 may be stored with a clinical profile that indicates whether the patient has diabetes by searching a patient's problem list for diabetes codes, searching the patient's medical list for insulin codes, searching the patient's most recent lab report for increased glucose levels, etc. Accordingly, the profile generator device 125 may include various mechanisms to intelligently combine the statuses extracted from various information carriers.

In a particular example of maintaining clinical profiles for patients to be kept in the EMR for these patients, the profile generator device 125 may maintain one or more profile templates that each specify a list of phenotypes where each phenotype may be modelled as an ICD (International Classification of Diseases) code. For each phenotype that is specified, the profile generator device 125 may utilize a phenotype extraction engine to assess the status of the phenotype in a given patient based on the information from the various information sources.

The RIS 130 may be a system tracking information related to image orders encompassing a reason associated with the image order. The reason for the image order may encompass one or more ICD codes which may be entered by the physician using the physician device 105 (e.g., as part of a Computerized Physician Order Entry (CPOE) workflow step). As those skilled in the art will understand, the RIS 130 may provide various functionalities and provide features in receiving and forwarding image orders from referring physicians. Accordingly, the RIS 130 may be configured with a functionality of receiving image orders and forward the image order to a querying component in the system 100.

The exam normalizing device 135 may be a component that generates a semantically normalized reason for the image order. In a substantially similar manner as the profile generator device 125, the exam normalizing device 135 may provide a normalizing operation for the image orders. As not all image orders are provided to the RIS 130 using a CPOE workflow or in another standardized format, the image order may be narrative. Accordingly, the exam normalizing device 135 may semantically normalize a narrative content including a reason for the image order.

In a particular exemplary embodiment, the exam normalizing device 135 may be implemented using various natural language processing or learning techniques. In a first example, these techniques may be used for abbreviation disambiguation. As physicians may enter abbreviations for various reasons, the exam normalizing device 135 may detect abbreviations and replace the abbreviations with their corresponding expanded form (e.g., "Eval PE" may be replaced with "Evaluate for pulmonary embolism"). In a second example, these techniques may be used for sentence detection where the end of sentences are detected if there are multiple sentences. In a third example, these techniques may be used for concept extraction. Specifically, the exam normalizing device 135 may detect and extract concepts from a narrative text where the concepts may be residing in a background ontology. In a fourth example, these techniques may be used for negation detection. In a similar manner as detecting and extracting concepts, the exam normalizing device 135 may detect negations and determine which concepts are under its scope (e.g., "NegEx" may be used which is based on a list of negation keywords and a set of rules determining the scope of a negation in the context of a sentence).

By using these techniques, the natural language processing pipeline may extract concepts from sentences and determine how to normalize the concepts as the reason for the image order. The normalizing may be performed by using a list of concepts that is augmented with negation status which may be considered as a normalized reason for exam. For example, the Radiological Society of North America (RSNA) has released a normalized list of reasons for an image order which may be used by the exam normalizing device 135. The concepts may be extracted from the image order and may be mapped for normalization.

The PACS 140 may be a system that tracks and logs sequences and/or protocols that are used in image acquisition procedures. Accordingly, data related to selected protocols/sequences as determined by the image interpreter using the image interpreter device 115 and protocols/sequences actually used by the technician using the image capture device 117 may be received by the PACS 140 to perform this functionality. In this manner, imaging studies of image acquisition procedures may be consumed to track the sequences/protocols that have been used, opened, etc. As those skilled in the art will understand, the PACS 140 may provide various functionalities and provide features in receiving and forwarding information related to image acquisition procedures including the captured images. Accordingly, the PACS 140 may be configured with a functionality of receiving the information of the image acquisition procedures based on selections from the image interpreter device 115 and uses from the image capture device 117. In addition, the PACS 140 may forward requested information to a querying component in the system 100.

The sequence normalizing device 145 may be a component that generates a semantically normalized sequence use for image orders. The sequence normalizing device 145 may normalize sequences/protocols that are stored in the PACS 140 based on a standardized lexicon of sequence names (e.g., an externally maintained standard). The sequence normalizing device 145 may use heterogeneous factors to (1) characterize sequences (e.g., by means of a text header and physical acquisition parameters) and to (2) map this feature vector on the sequence lexicon. This mapping may be based on a local mapping table maintained at the medical institution or may be based on predetermined operations that intelligently parse out elements from the sequence header name (e.g., anatomy). The normalization performed by the sequence normalizing device 145 may be used to compare identical sequences across modalities that are labelled differently.

The workflow server 150 may be a component of the system 100 that performs functionalities associated with determining the sequences and/or protocols that are to be used for an image acquisition procedure according to the exemplary embodiments. As will be described in further detail below, the workflow server 150 may include a first mechanism in which information from past image acquisition procedures and corresponding image orders are used to generate parameters or rules that define when a particular sequence or protocol is to be used in image acquisition procedures for subsequent image orders. Accordingly, data may be requested from the medical data repository 120, the RIS 130, and/or the PACS 140. The workflow server 150 may include a second mechanism in which a new image order is received and sequences and/or protocols to be used in the image acquisition procedure are determined. Accordingly, when the image interpreter device 115 is being used to determine the sequences/protocols to be used for an image order, the workflow server 150 may provide outputs that automatically indicate the selections or provide a recommendation for the selections.

It is noted that the system 100 may include a plurality of physician devices 105, a plurality of image interpreter devices 115, a plurality of image capture devices 117, and a plurality of workflow servers 150. That is, many different physicians and image interpreters may utilize the system 100. There may also be many different workflow servers 150 that service different physician devices 105 and image interpreter devices 115. The medical data repository 120, the RIS 130, and the PACS 140 may also be systems including a plurality of different components (e.g., the profile generator device 125, the exam normalizing device 135, and the sequence normalizing device, respectively).

It is also noted that the functionalities of the medical data repository 120, the RIS 130, and the PACS 140 along with the profile generator device 125, the exam normalizing device 135, and the sequence normalizing device 145 being implemented in separate components of the system 100 is only exemplary. According to another exemplary embodiment, the medical data repository 120 may incorporate the functionality of the profile generator device 125, the RIS 130 may incorporate the functionality of the exam normalizing device 135, and the PACS 140 may incorporate the functionality of the sequence normalizing device 145. According to a further exemplary embodiment, the workflow server 150 may incorporate the functionalities of the profile generator device 125, the exam normalizing device 135, and the sequence normalizing device 145. Thus, the workflow server 150 may receive any type of data from the medical data repository 120, the RIS 130, and the PACS 140. According to yet another exemplary embodiment, the workflow server 150 may incorporate all the functionalities of the medical data repository 120, the profile generator device 125, the RIS 130, the exam normalizing device 135, the PACS 140, and the sequence normalizing device 145.

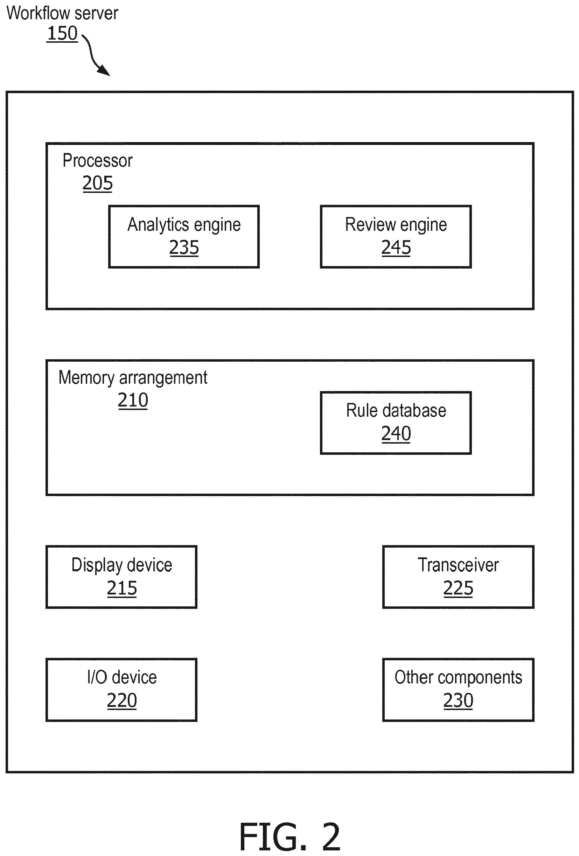

As described above, the workflow server 150 may generate rules by which selections of sequences/protocols may be made based on prior image acquisition procedures. Fig. 2 shows the workflow server 150 of Fig. 1 according to the exemplary embodiments. The workflow server 150 may provide various functionalities in determining the selections of the sequences/protocols for an image acquisition procedure. Although the workflow server 150 is described as a network component (specifically a server), the workflow server 150 may be embodied in a variety of hardware components such as a portable device (e.g., a tablet, a smartphone, a laptop, etc.), a stationary device (e.g., a desktop terminal), incorporated into the physician device 105 and/or the image interpreter device 115, incorporated into a website service, incorporated as a cloud device, etc. The workflow server 150 may include a processor 205, a memory arrangement 210, a display device 215, an input and output (I/O) device 220, a transceiver 225, and other components 230 (e.g., an imager, an audio I/O device, a battery, a data acquisition device, ports to electrically connect the workflow server 150 to other electronic devices, etc.).

The processor 205 may be configured to execute a plurality of applications of the workflow server 150. As will be described in further detail below, the processor 205 may utilize a plurality of engines including an analytics engine 235 and a review engine 245. The analytics engine 235 may be utilized for operations associated with the first mechanism of generating the rules. Specifically, the analytics engine 235 may be configured to determine how sequences/protocols have been used in image acquisition procedures and generate appropriate rules. The review engine 245 along with the rules generated by the analytics engine 235 may be utilized for operations associated with the second mechanism of processing an image order and determining the sequences/protocols to be used. Specifically, the review engine 245 may be configured to process an image order and determine the sequences/protocols that are to be used.

It should be noted that the above noted applications and engines each being an application (e.g., a program) executed by the processor 205 is only exemplary. The functionality associated with the applications may also be represented as components of one or more multifunctional programs, a separate incorporated component of the workflow server 150 or may be a modular component coupled to the workflow server 150, e.g., an integrated circuit with or without firmware.

The memory 210 may be a hardware component configured to store data related to operations performed by the workflow server 150. Specifically, the memory 210 may store data related to the engines 235, 245 such as the image order and the parameters thereof. The memory 210 may also store the rules generated by the analytics engine 235 in a rules database 240 that may be used by the review engine 245. The display device 215 may be a hardware component configured to show data to a user while the I/O device 220 may be a hardware component that enables the user to enter inputs. For example, an administrator of the workflow server 150 may maintain and update the functionalities of the workflow server 150 through user interfaces shown on the display device 215 with inputs entered with the I/O device 220. It should be noted that the display device 215 and the I/O device 220 may be separate components or integrated together such as a touchscreen. The transceiver 225 may be a hardware component configured to transmit and/or receive data via the communications network 110.

According to the exemplary embodiments, the workflow server 150 may perform various different operations to determine the sequences/protocols that are to be used for an image order by a technician using the image capture device 117. Initially, as described above, the analytics engine 235 may be configured to determine how sequences/protocols have been used in image acquisition procedures and generate appropriate rules. Specifically, the analytics engine 235 may analyze a relevance of sequences and/or protocols that were selected for use in previous image acquisition procedures based on a context in which the image acquisition procedures were performed. In evaluating the previous image acquisition procedures, the analytics engine 235 may track the sequences and/or protocols that are used across image orders from referring physicians and selections from image interpreters for patients. As noted above, the analytics engine 150 may request the appropriate data to perform this functionality from the medical data repository 120, the RIS 130, and the PACS 140.

The analytics engine 235 may determine key metrics associated with a particular sequence or protocol as related to a context (e.g., a given reason) of the image order from which the image acquisition procedure was performed. For example, a sequence or protocol may be analyzed based on its relevance to a specific reason. Specifically, the analytics engine 235 may use the information from the medical data repository 120, the RIS 130, and the PACS 140 to determine a percentage of the image acquisition procedures in which a sequence or protocol was used to acquire an image. The analytics engine 235 may also use the information from the medical data repository 120, the RIS 130, and the PACS 140 to determine a percentage of interpretations where an image captured with the sequence or protocol was opened. Based on this ratio, the analytics engine 235 may provide an indication of a priority or necessity of the sequence or protocol to the given reason for the image order. For example, based on a scale of 0 to 100, if the ratio of the percentages is close to or equal to 0 or a low value (e.g., 0 to 32), the indication may be that the sequence or protocol is a candidate for elimination when the given reason arises in an image order. In another example, if the ratio of the percentages is an intermediary value (e.g., 33 to 66), the indication may be that the sequence or protocol is a candidate for being suggested but not required when the given reason arises in an image order. In a further example, if the ratio of the percentages is a high value (e.g., 67 to 100), the indication may be that the sequence or protocol is a candidate for being required when the given reason arises in an image order.

In a specific example of the above priority determination, a protocol may include a first, second, and third sequence. The protocol may have been selected for use in an image acquisition protocol based on characteristics or parameters of an image order. However, the characteristics of the image order may be used to identify that the first sequence is mandatory, the second sequence is suggested, and the third sequence is eliminated. In this manner, when the protocols/sequences for the image acquisition procedure are eventually selected, the first sequence may be included, the third sequence may be omitted, and the second sequence may be included at the discretion of the image interpreter selecting the protocols/sequences for the image acquisition procedure corresponding to the image order.

In addition to the metrics described above, the analytics engine 235 may be configured with further operations to analyze the sequence/protocol being used for a given reason of an image order. Specifically, the further operations may be used to research any patterns that may underlie the sequence/protocol being analyzed. For example, a pattern may suggest that a particular sequence is infrequently used or an image captured with the particular sequence is infrequently opened. The analytics engine 235 may determine the patterns at the level of the clinical profile of the EMR in the medical data repository 120 and/or the properties or parameters of the image order.

The further operations of the analytics engine 235 may be implemented in various ways. In a first example, the analytics engine 235 may display relevant information for each investigated sequence/protocol (e.g., sequence or protocol name, identity of the referring physician or image interpreter, reason for the image order, acquisition modality name, patient clinical profile, etc.). In a second example, the analytics engine 235 may provide an analytics front end (e.g., a filter system) to investigate for a given sequence or protocol with regard to distribution. Specifically, the investigation may be for a distribution across reasons for image orders, across time, grouped by reading image interpreter, etc. In a third example, the analytics engine 235 may group relevant information and provide statistics corresponding thereto. In this manner, the analytics engine 235 may show how a particular sequence or protocol was related to a given reason (e.g., an image captured with a particular sequence is opened 10 of 15 times in image acquisition procedures with "headache" as the reason for the image orders). In a fourth example, the analytics engine 235 may group relevant information based on hierarchical reasoning. Specifically, the analytics engine 235 may traverse concepts in an ontology from specific to general to determine how a sequence or protocol is used for a particular reason (e.g., find that 13 of 15 times an image captured with a particular sequence was opened for cancer patients).

The analytics engine 235 may create and edit if-then rules that populate the rule database 240 that suggest to (or not) acquire an image with a particular sequence or protocol in the context of a given protocol (for sequences), image order, reason for the image order, clinical profile, etc. The analytics engine 235 may further be configured to detect discrepancies between image interpreters and specifications of protocols or sequences so that the rules being generated remain consistent.

The rule database 240 may be configured to store the rules generated or updated by the analytics engine 235 for use by the review engine 245. Specifically, once stored, the review engine 245 may use the rules to determine or suggest to include or exclude a sequence or protocol from being used in an image acquisition procedure for an image order. Again, the rules may be used determine or suggest a sequence or protocol based on the context (e.g., a protocol, an image order, a reason for the image order, clinical profile, etc.) of the image order.

Initially, it is noted that the rule database 240 being a database stored in the memory 210 is only exemplary. The rule database 240 may represent any data repository in which the rules that are generated and used by the workflow server 150 are stored. According to another exemplary embodiment, the rule database 240 may be implemented as a separate rule repository (not shown) where the workflow server 150 may be communicatively connected to access the data therein.

The rules stored in the rule database 240 may be labeled in different manners. As noted above, the analytics engine 235 may determine a priority or necessity of the rules. Accordingly, the rules may be labeled, for example, as mandatory, suggested, or eliminated. When the rule indicates that a particular sequence or protocol is mandatory or eliminated for a given context, the rule may be one that must be adhered. For example, for a first context, a rule may indicate that a particular sequence is mandatory such that the sequence is included in the selections for the image acquisition procedure. In another example, for a second context, a rule may indicate that a particular sequence is eliminated such that a protocol including the sequence is updated to remove this sequence from the image acquisition procedure. When the rule indicates that a particular sequence or protocol is suggested for a given context, the rule may be one that is to be considered (e.g., based on an acknowledgement from the image interpreter). For example, for a given context, a rule may indicate that a particular protocol may be used to capture images that provide medically relevant images such that an indication from the image interpreter may be the decision used to include or exclude the protocol for this image acquisition procedure.

The rules stored in the rule database 240 may include further types of rules that are not based solely on the determinations of the analytics engine 235. In a first example, in addition to the automatically generated rules by the analytics engine 235, the rules may include one or more user-modified or user-specified rules. As noted above, an administrator may use the display device 215 and the I/O device 220 to provide inputs for various reasons. One reason may include adding a manually created rule, removing an automatically generated rule, or modifying a rule already stored in the rule database 240. In this manner, the rule database 240 may further allow for this modification to the rules.

In a second example, the rules may include a comparison rule that leverages a comparison for a new image order of a patient with prior image acquisition procedures performed on the patient. This comparison may be used to determine a correspondence between the new image order and at least one of the prior image acquisition procedures and the protocols/sequences that were used. Accordingly, the process of determining the protocols/sequences to be used in the image acquisition procedure for the new image order may be performed more efficiently by referencing the selections of the protocols/sequences used in a properly matched prior image acquisition procedure.

For example, if the reason for a new image order is identical or substantially similar to the reason for a prior image acquisition order corresponding to a prior image order associated with the patient (e.g., a most recent imaging study), the rules may then be used to identify if the prior image acquisition order with its matched reason also has a matching anatomy and modality to the new image order. In such a scenario, the rule may suggest to only acquire images using the protocols/sequences that were used as part of the prior matching image acquisition procedure. This rule may be further refined to omit those protocols/sequences that were selected in the prior matching image acquisition procedure if the image interpreter did not later use the corresponding image during the interpretation aspect of the image acquisition procedure. That is, there may be sequences or protocols which may not have been used the prior time. Furthermore, the omission may be due to any reason that warrants the removal from the selections in the new image order (e.g., because the omitted sequence is only used in an initial image acquisition procedure and is not needed for a following image acquisition procedure with matching characteristics).

In a third example, the rules may include one or more safety rules. The safety rules may be a particular example of the user-specified rules that are specifically directed toward safety protocols. For example, there may be a safety protocol where a patient with a known condition (e.g., kidney failure as may be indicated in the clinical profile in the EMR of the patient stored in the medical data repository 120) should not have a particular sequence used to capture an image (e.g., a contrast sequence). In this manner, the rule database 240 may include safety rules to ensure that certain protocols/sequences are only included until the conditions of the patient are first considered.

In a fourth example, the rules may include one or more compliance or regulatory rules. The compliance rules may be another particular example of the user-specified rules that are specifically directed toward compliance and regulatory protocols (e.g., as set by a regulatory agency, federal standards entity, third party such as an insurance carrier, etc.). In this manner, the rule database 240 may include compliance rules to ensure that legal and exterior considerations are incorporated into the selection process of protocols and sequences in an image acquisition procedure corresponding to an image order.

It is noted that the rule database 240 may be dynamically updated such that the rules stored therein are not comprehensive of all rules that have been included but includes only the rules that are to be applied for incoming image orders. For example, when new research has definitely shown the need to include or exclude a particular sequence in an overall manner or for a given context, a corresponding rule may override all other rules for this sequence. In another example, when the rules are known to change over time, rules that have been in effect for a particular time period may be reviewed to be kept, be eliminated for being outdated, or may be used but with an alert indicating the possibility of being outdated.

The review engine 245 may be configured to process an image order and determine the sequences/protocols that are to be used. Specifically, the review engine 245 may be used to provide information for selections of protocols and/or sequences to the image interpreter device 115 such that an image acquisition procedure for an image order may be planned accordingly. According to an exemplary embodiment, the review engine 245 may provide a user interface to the image interpreter device 115 that includes determinations of protocols and sequences that are to be used based on operations of the review engine 245 using a received image order and the rules in the rule database 240. Specifically, characteristics of the received image order may be determined by the review engine 245 to determine the context. Based on this context, the rules corresponding to this context as stored in the rule database 240 may be used in determining which of the protocols and/or sequences are to be used in an image acquisition procedure for this image order having the determined context.

The interface may enable the image interpreter to review the image order and proceed in different manners. In a first manner, the image interpreter may manually select protocols and/or sequences to be used for the resulting image acquisition procedure. Thereafter, the review engine 245 may determine if there are to be any modifications to the selected protocols/sequences (e.g., addition, deletion, or change). For example, there may be a missing mandatory protocol which may be added. In another example, there may be an unnecessary sequence that was selected which may be omitted. In a second manner, the image interpreter may request the protocols and/or sequences from the review engine 245 that are to be included in the resulting image acquisition procedure. In either case, the image interpreter may utilize an automated feature to accept the output from the workflow server 150 or may utilize a manual feature to make all final decisions regarding the selections of the protocols/sequences. As noted above, when the manual feature is used, the image interpreter may also be provided with a priority indication (e.g., mandatory, suggested, and eliminated) so that the image interpreter may make a further informed decision.

It is noted that in a particular exemplary embodiment, the selection of the protocols and/or sequences as determined by the workflow server 150 may be automatically applied. For example, there may be rules that indicate certain protocols or sequences that are mandatory for a given context (e.g., having a score near 100) and are included without any user intervention. In another example, there may be rules that indicate certain protocols or sequences that are to be eliminated for a given context (e.g., having a score near 0) and are omitted without any user intervention.

Once the selections of the protocols and/or sequences have been made by the image interpreter for the image order, an exam card may be created for the corresponding image acquisition procedure. This exam card may be provided to the image capture device 117 which may be directly consumed after the technician has selected the patient from a modality worklist. This streamlined process may reduce time in terms of preparing the modality of the examination and eliminate incorrectly selected protocols.

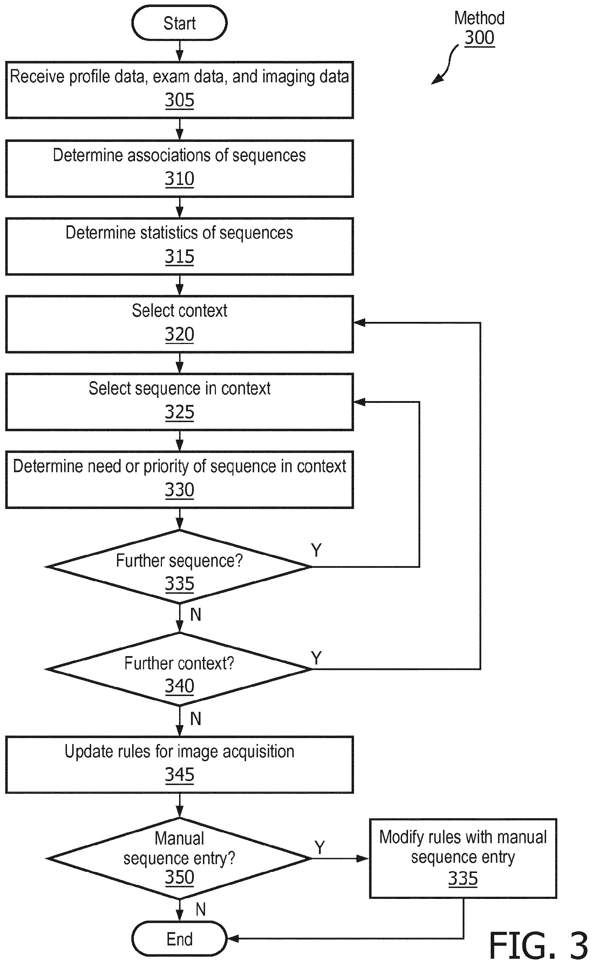

Fig. 3 shows a method 300 for managing protocols and sequences of the protocols according to the exemplary embodiments. Specifically, the method 300 may relate to the first mechanism of the exemplary embodiments in which the analytics engine 235 for the workflow server 150 determines the rules to be used in processing image orders based on information of previous image acquisition procedures. Accordingly, the method 300 will be described from the perspective of the workflow server 150. The method 300 will also be described with regard to the system 100 of Fig. 1 and the workflow server 150 of Fig. 2.

In 305, the workflow server receives data from the medical data repository 120, the RIS 130, and the PACS 140. In this manner, profile data may be received that relates to clinical profiles in a corresponding EMR of respective patients. Exam data may also be received that relates to reasons for which image orders are scheduled for the patients. In this manner, a context for the image orders and the image acquisition procedures may be identified. Imaging data may further be received that relates to how protocols and/or sequences are used in the image acquisition procedures. Although the description above and the exemplary embodiments relate to determining protocols and/or sequences, for illustrative purposes, the method 300 is described with regard to sequences only. However, the method 300 may also be used for protocols. As noted above, the data may have been normalized in some manner using the profile generator device 125, the exam normalizing device 135, and the sequence normalizing device 145. Once normalized, connections among the data may be identified in a more standardized format. Accordingly, in 310, the workflow server 150 determines the associations of the sequences to the patient and/or the context.

In 315, the workflow server 150 determines statistics of the sequences based on a percentage of times that a particular sequence is selected for use in the capture phase of the image acquisition procedure to capture an image as well as a percentage of times that a resulting captured image from using the particular sequence is used in the interpretation phase of the image acquisition procedure. The statistics may be used to identify when a sequence is considered mandatory, suggested, or eliminated for a given context or in an overall consideration (e.g., eliminated from all contexts). It is noted that the statistics is only exemplary and as described in detail above, the workflow server 150 may utilize any metric or value in determining how a sequence was used for a selected context.

In 320, the workflow server 150 selects a context for an image order. As noted above, the context may be based on various considerations such as a protocol name, an identity of a referring physician, an identity of an image interpreter, a reason for the image order, an acquisition modality, the EMR of the patient, a combination thereof, etc. In 325, the workflow server 150 selects a sequence in the context. As noted above, an image acquisition procedure (as related to a context) may use one or more protocols which may include one or more sequences. In 330, the workflow server 150 determines a need or priority of the sequence given the context. For example, the priority may indicate that the sequence is mandatory, suggested, or eliminated for the given context.

In 335, the workflow server 150 determines whether there is a further sequence in the context. If there is a further sequence, the workflow server 150 returns to 325. This process may continue until all the sequences for the given context are analyzed for priority.

When the sequences for a selected context have been analyzed, further contexts may be analyzed as well. In 340, the workflow server 150 determines whether there is a further context. If there is a further context, the workflow server 150 returns to 320. This process may continue until all contexts and all included sequences are analyzed.

In 345, the workflow server 150 updates the rules in the rule database 240 based on the determinations made above. As described above, the rules may be added, removed, or changed. In this manner, the rules may be available for use by the review engine 245.

As noted above, the rule database 240 may include various types of rules. The above mechanism describes how automatically generated rules are created based on the data from the various sources. However, there may be other rules such as user-generated rules, safety rules, compliance rules, etc. The automatically generated rules may also be changed as user-modified rules. In 350, the workflow server 150 determines whether there has been any manual sequence entry or change thereto. If there is a manual entry, in 355, the workflow server 150 modifies the rules based on the manual sequence entry.

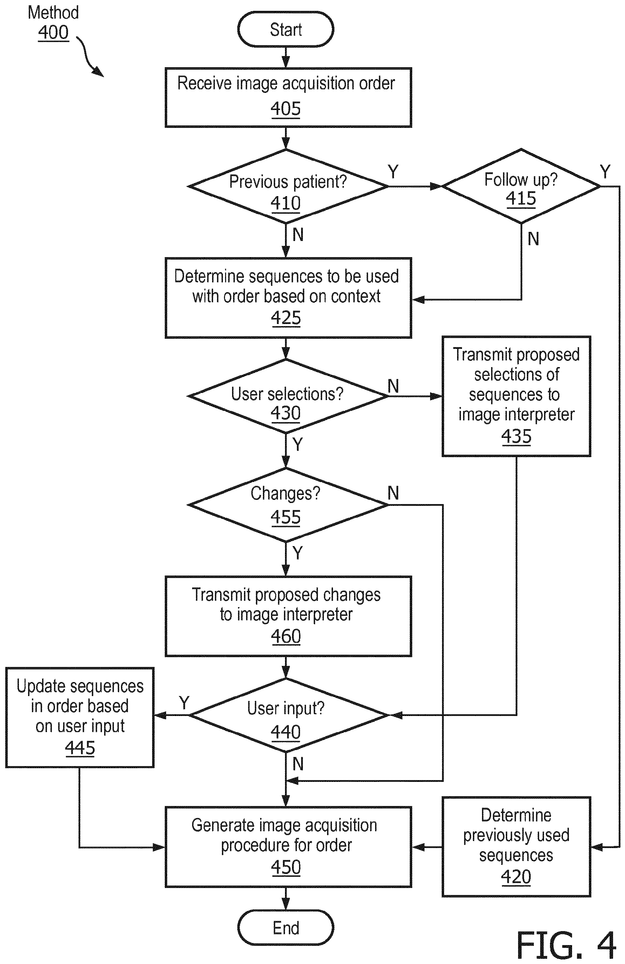

Fig. 4 shows a method 400 for selecting a protocol and/or a sequence for an image order according to the exemplary embodiments. Specifically, the method 400 may relate to the second mechanism of the exemplary embodiments in which the analytics engine 235 for the workflow server 150 determines the rules to be used in processing image orders based on information of previous image acquisition procedures. Accordingly, the method 300 will be described from the perspective of the workflow server 150. The method 300 will also be described with regard to the system 100 of Fig. 1 and the workflow server 150 of Fig. 2.

In 405, the workflow server 150 receives an image acquisition order from a referring physician. As described above, the physician using the physician device 105 may submit an image order for a patient upon whom an image acquisition procedure is to be performed. The image order may include various types of information which may be used in identifying a context for the image order. For example, the image order may include a reason which may be used in determining the context.

In 410, the workflow server 150 determines an identity of the patient associated with the image order. If the image order relates to a previous patient or a patient who has information available in the medical data repository 120, the RIS 130, and/or the PACS 140, the workflow server 150 may then determine if the image order relates to a follow up visit for the patient. That is, the follow up visit may represent any subsequent image acquisition procedure having identical or substantially similar parameters to a prior image acquisition procedure. There may be further considerations as to whether the image order is for a follow up visit. For example, the follow up visit may be determined if the image order is within a predetermined time frame from the previous image order. If the image order is for a follow up visit of a previous patient, in 420, the workflow server determines previously used sequences that were selected and used for the prior image acquisition procedure. Similar to the method 300, although the description above and the exemplary embodiments relate to determining protocols and/or sequences, for illustrative purposes, the method 400 is described with regard to sequences only. However, the method 400 may also be used for protocols. It is noted that the method 400 may include additional operations. For example, when the previously used sequences are to be refined in view of the image order being for a follow up visit.

When the image order is not for a previous patient or when the image order is for a previous patient but not for a follow up visit, the workflow server 150 continues from 410 or 415 to 425. In 425, the workflow server 150 determines the sequences to be used with the image order based on the context of the received image order. Specifically, the context of the received image order in 405 may be used to compare to contexts in the rule database 240. By comparing the context of the received image order to previous contexts, the associated rules for the context may be identified and used to determine which of the sequences are to be included in an image acquisition procedure for the received image order.

In 430, the workflow server 150 determines whether the image interpreter has already made selections for sequences to be used in the image acquisition procedure. As noted above, the features of the workflow server 150 may be used in a variety of manners. In a first example, the image interpreter may select sequences and receive an output from the workflow server 150 as to whether the selections are optimal. In a second example, the image interpreter may request the workflow server 150 to provide suggested sequences as determined in 425.

If the image interpreter requests that the determined sequences be provided, the workflow server 150 continues to 435. In 435, the workflow server 150 transmits the proposed selections of sequences to the image interpreter. It is again noted that the proposed selections may be provided with indicia that identify whether a proposed selection is for a sequence that is mandatory, suggested, or eliminated. In 440, the image interpreter may review the selections of the sequences as determined by the workflow server 150 and determine if there are to be any changes. For example, the image interpreter may wish to add a sequence that was not suggested or remove a sequence that was included. In another example, when there are suggested but not mandatory sequences, the image interpreter may be requested to select whether to include or exclude these sequences from the image acquisition procedure. If there are no inputs from the image interpreter, the workflow server 150 continues to 450. However, if there are inputs from the image interpreter, the workflow server 150 continues to 445 where the sequences are updated to incorporate the inputs. Thus, in 450, the workflow server 150 generates the image acquisition procedure for the image order.

Returning to 430, if the image interpreter has made selections for sequences, the workflow server 150 continues to 440. In 455, the workflow server 150 determines whether there are changes to be applied or recommended to the selections of the image interpreter. For example, the selections may have omitted a mandatory sequence or included an outdated sequence that should be omitted. If the selections by the image interpreter match the sequences determined in 425, the workflow server 150 continues to 450. However, if the selections by the image interpreter require changes to be made, the workflow server continues to 460. In 460, the workflow server 150 transmits proposed changes to the selections made by the image interpreter. Thus, in 440, any inputs from the image interpreter based on the proposed changes may be incorporated. It is again noted that the proposed changes may be provided with indicia that identify whether a proposed change is for a sequence that is mandatory, suggested, or eliminated.

The exemplary embodiments provide a device, system, and method of optimizing an image acquisition workflow by determining which sequences or protocols to be used in an image acquisition procedure that minimizes or eliminates waste. Specifically, based on rules generated using historical information of previous image acquisition procedures and incorporating other rules, the mechanism according to the exemplary embodiments may include sequences/protocols that are known to have a high probability of capturing medically relevant images (e.g., always used during an interpretation) and omit sequences/protocols that are known to have a low probability of capturing medically relevant images (e.g., never used during an interpretation). Therefore, the exemplary embodiments may allow a technician to perform the image acquisition procedure with only those sequences/protocols that are of utility. In this manner, no additional time is required by the technician in performing the image capturing portion of the image acquisition procedure and no wasted time is created for the image interpreter in performing the interpretation portion of the image acquisition procedure. Furthermore, the referring physician who scheduled the image order may also receive the results in a timelier manner.

Those skilled in the art will understand that the above-described exemplary embodiments may be implemented in any suitable software or hardware configuration or combination thereof. An exemplary hardware platform for implementing the exemplary embodiments may include, for example, an Intel x86 based platform with compatible operating system, a Windows platform, a Mac platform and MAC OS, a mobile device having an operating system such as iOS, Android, etc. In a further example, the exemplary embodiments of the above described method may be embodied as a computer program product containing lines of code stored on a computer readable storage medium that may be executed on a processor or microprocessor. The storage medium may be, for example, a local or remote data repository compatible or formatted for use with the above noted operating systems using any storage operation.

## Claims

1. A method, comprising:
at a workflow server:
receiving data associated with previous image acquisition procedures that have been performed, the image acquisition procedures having respective image orders, the image orders having respective contexts, each of the previous image acquisition procedures having used at least one sequence defining settings to capture an image;
determining at least one metric associated with a use of a first one of the at least one sequence for a first one of the contexts;
generating a rule indicative of whether the first sequence is to be included or excluded for a subsequent image acquisition procedure having the first context based on the at least one metric;
receiving a subsequent image order corresponding to the subsequent image acquisition procedure, the subsequent image order having a subsequent context;
determining whether the subsequent context corresponds to the first context; and
when the subsequent context corresponds to the first context, determining an indication of whether the first sequence is to be included or excluded in the subsequent image acquisition procedure based on the rule;
wherein the at least one metric comprises a percentage of image captures in the previous acquisition procedures for the first context in which the first sequence is used, a percentage of interpretations in the previous acquisition procedures for the first context in which the image captures are used, or a combination thereof;
wherein the method further comprises automatically implementing the indication for the first sequence in the subsequent image acquisition procedure.

2. The method of claim 1, wherein the data comprises at least one electronic medical record (EMR), image order information encompassing a reason for an image order, sequence use information, or a combination thereof.

3. The method of claim 1, wherein the rule identifies a priority of the first sequence for the first context, wherein the priority is whether the first sequence is mandatory for the first context, suggested for the first context, or is to be omitted for the first context.

4. The method of claim 1, wherein the first context is based on a first protocol that includes the first sequence, a first image order corresponding to a first image acquisition procedure, a first reason for the first image order, a clinical profile of at least one patient who has had the first image acquisition procedure, or a combination thereof.

5. The method of claim 1, further comprising:
receiving a manual entry for a further rule, wherein the further rule is one of a user-modified rule, a user-generated rule, a safety rule, a compliance rule, or a combination thereof.

6. The method of claim 1, wherein the image acquisition procedures correspond to image capturing operations performed with an X-ray procedure, a computed tomography (CT) procedure, a magnetic resonance imaging (MRI) procedure, an ultrasound procedure, a positron emission tomography (PET) scan procedure, a single-photon emission computed tomography (SPECT) scan procedure, or a combination thereof.

7. A workflow server, comprising:
a transceiver communicating via a communications network, the transceiver configured to receive data associated with previous image acquisition procedures that have been performed, the image acquisition procedures having respective image orders, the image orders having respective contexts, each of the image acquisition procedures having used at least one sequence defining settings to capture an image;
a memory storing an executable program; and
a processor that executes the executable program that causes the processor to perform operations, comprising:
determining at least one metric associated with a use of a first one of the at least one sequence for a first one of the contexts;
generating a rule indicative of whether the first sequence is to be selected for a subsequent image acquisition procedure having the first context based on the at least one metric;
receiving a subsequent image order corresponding to the subsequent image acquisition procedure, the subsequent image order having a subsequent context;
determining whether the subsequent context corresponds to the first context; and
when the subsequent context corresponds to the first context, determining an indication of whether the first sequence is to be included or excluded in the subsequent image acquisition procedure based on the rule; and automatically implementing the indication for the first sequence in the subsequent image acquisition procedure;
wherein the at least one metric comprises a percentage of image captures in the previous acquisition procedures for the first context in which the first sequence is used, a percentage of interpretations in the previous acquisition procedures for the first context in which the image captures are used, or a combination thereof.

8. The workflow server of claim 8, wherein the rule identifies a priority of the first sequence for the first context, wherein the priority is whether the first sequence is mandatory for the first context, suggested for the first context, or is to be omitted for the first context.

9. The workflow server of claim 8, wherein the first context is based on a first protocol that includes the first sequence, a first image order corresponding to a first image acquisition procedure, a first reason for the first image order, a clinical profile of at least one patient who has had the first image acquisition procedure, or a combination thereof.

10. The workflow server of claim 8, wherein the transceiver further receives a manual entry for a further rule, wherein the further rule is one of a user-modified rule, a user-generated rule, a safety rule, a compliance rule, or a combination thereof.

## Patentansprüche

1. Verfahren, umfassend: auf einem Workflow-Server:
empfangen von Daten, die mit vorherigen Bilderfassungsverfahren verknüpft sind, die durchgeführt wurden, wobei die Bilderfassungsverfahren entsprechende Bildreihenfolgen haben, die Bildreihenfolgen entsprechende Kontexte haben und jedes der vorherigen Bilderfassungsverfahren mindestens eine Sequenz verwendet hat, die Einstellungen zum Erfassen eines Bildes definiert;
bestimmen mindestens einer Metrik, die mit einer Verwendung einer ersten der mindestens eine Sequenz für einen ersten der Kontexte verbunden ist; erzeugen einer Regel, die angibt, ob die erste Sequenz für einen nachfolgenden Bildaufnahmevorgang mit dem ersten Kontext basierend auf der mindestens eine Metrik eingeschlossen oder ausgeschlossen werden soll;
empfangen einer nachfolgenden Bildreihenfolge, die der nachfolgenden Bildaufnahmeprozedur entspricht, wobei die nachfolgende Bildreihenfolge einen nachfolgenden Kontext aufweist; bestimmen, ob der nachfolgende Kontext dem ersten Kontext entspricht; und wenn der nachfolgende Kontext dem ersten Kontext entspricht, Bestimmen eines Hinweises darauf, ob die erste Sequenz basierend auf der Regel in die nachfolgende Bilderfassungsprozedur einbezogen oder ausgeschlossen werden soll; wobei die mindestens eine Metrik einen Prozentsatz von Bilderfassungen in den vorherigen Erfassungsverfahren für den ersten Kontext, in dem die erste Sequenz verwendet wird, einen Prozentsatz von Interpretationen in den vorherigen Erfassungsverfahren für den ersten Kontext, in dem die Bilderfassungen verwendet werden, umfasst, oder eine Kombination davon;
wobei das Verfahren weiterhin das automatische Implementieren der Angabe für die erste Sequenz im nachfolgenden Bildaufnahmevorgang umfasst.

2. Verfahren nach Anspruch 1, wobei die Daten mindestens eine elektronische Krankenakte (EMR), Informationen zur Bildbestellung, die einen Grund für eine Bildbestellung umfassen, Informationen zur Sequenzverwendung oder eine Kombination davon umfassen.

3. Verfahren nach Anspruch 1, wobei die Regel eine Priorität der ersten Sequenz für den ersten Kontext identifiziert, wobei die Priorität darin besteht, ob die erste Sequenz für den ersten Kontext obligatorisch ist, für den ersten Kontext vorgeschlagen wird oder weggelassen werden soll der erste Kontext.

4. Verfahren nach Anspruch 1, wobei der erste Kontext auf einem ersten Protokoll basiert, das die erste Sequenz, eine erste Bildreihenfolge entsprechend einem ersten Bildaufnahmeverfahren, einen ersten Grund für die erste Bildreihenfolge, ein klinisches Profil von at umfasst mindestens ein Patient, bei dem das erste Bildaufnahmeverfahren durchgeführt wurde, oder eine Kombination davon.

5. Verfahren nach Anspruch 1, weiterhin umfassend: empfangen eines manuellen Eintrags für eine weitere Regel, wobei die weitere Regel eine vom Benutzer geänderte Regel, eine vom Benutzer generierte Regel, eine Sicherheitsregel, eine Compliance-Regel oder eine Kombination davon ist.

6. Verfahren nach Anspruch 1, wobei die Bilderfassungsprozeduren Bildaufnahmeoperationen entsprechen, die mit einem Röntgenverfahren, einem Computertomographie (CT)-Verfahren, einem Magnetresonanztomographie (MRI)-Verfahren, einem Ultraschallverfahren, einem Positronenemissionstomographie (PET)-Scanverfahren, einem Einzelphotonenemissions-Computertomographie (SPECT)-Scanverfahren oder einer Kombination davon durchgeführt werden.

7. Workflow-Server, umfassend: einen Transceiver, der über ein Kommunikationsnetzwerk kommuniziert, wobei der Transceiver so konfiguriert ist, dass er Daten empfängt, die mit vorherigen Bilderfassungsprozeduren verknüpft sind, die durchgeführt wurden, wobei die Bilderfassungsprozeduren entsprechende Bildreihenfolgen haben, die Bildbestellungen entsprechende Kontexte haben und jede der Bilderfassungsprozeduren verwendet wurde mindestens eine Sequenz, die Einstellungen zum Erfassen eines Bildes definiert; einen Speicher, der ein ausführbares Programm speichert; und einen Prozessor, der das ausführbare Programm ausführt, das den Prozessor veranlasst, Operationen auszuführen, umfassend: bestimmen mindestens einer Metrik, die mit einer Verwendung einer ersten der mindestens eine Sequenz für einen ersten der Kontexte verbunden ist; erzeugen einer Regel, die angibt, ob die erste Sequenz für eine nachfolgende Bilderfassungsprozedur mit dem ersten Kontext basierend auf der mindestens eine Metrik ausgewählt werden soll; empfangen einer nachfolgenden Bildreihenfolge, die der nachfolgenden Bildaufnahmeprozedur entspricht, wobei die nachfolgende Bildreihenfolge einen nachfolgenden Kontext aufweist; bestimmen, ob der nachfolgende Kontext dem ersten Kontext entspricht; und wenn der nachfolgende Kontext dem ersten Kontext entspricht, Bestimmen eines Hinweises darauf, ob die erste Sequenz basierend auf der Regel in die nachfolgende Bilderfassungsprozedur einbezogen oder ausgeschlossen werden soll; und automatisches Implementieren der Angabe für die erste Sequenz im nachfolgenden Bildaufnahmevorgang; wobei die mindestens eine Metrik einen Prozentsatz von Bilderfassungen in den vorherigen Erfassungsverfahren für den ersten Kontext, in dem die erste Sequenz verwendet wird, einen Prozentsatz von Interpretationen in den vorherigen Erfassungsverfahren für den ersten Kontext, in dem die Bilderfassungen verwendet werden, umfasst, oder eine Kombination davon.

8. Workflow-Server nach Anspruch 8, wobei die Regel eine Priorität der ersten Sequenz für den ersten Kontext identifiziert, wobei die Priorität darin besteht, ob die erste Sequenz für den ersten Kontext obligatorisch ist, für den ersten Kontext empfohlen wird oder weggelassen werden soll für den ersten Kontext.

9. Workflow-Server nach Anspruch 8, wobei der erste Kontext auf einem ersten Protokoll basiert, das die erste Sequenz, eine erste Bildreihenfolge, die einem ersten Bildaufnahmeverfahren entspricht, einen ersten Grund für die erste Bildreihenfolge und ein klinisches Profil enthält mindestens ein Patient, der sich dem ersten Bildaufnahmeverfahren unterzogen hat, oder eine Kombination davon.

10. Workflow-Server nach Anspruch 8, wobei der Transceiver weiterhin einen manuellen Eintrag für eine weitere Regel empfängt, wobei die weitere Regel eine von einer benutzermodifizierten Regel, einer benutzergenerierten Regel, einer Sicherheitsregel, einer Compliance-Regel oder dergleichen ist eine Kombination davon.

## Revendications

1. Procédé comprenant: sur un serveur de flux de travail: recevoir des données associées à des procédures d'acquisition d'images précédentes qui ont été effectuées, les procédures d'acquisition d'images ayant des ordres d'images respectifs, les ordres d'images ayant des contextes respectifs, chacune des procédures d'acquisition d'images précédentes ayant utilisé au moins une séquence définissant des paramètres pour capturer une image; déterminer au moins une métrique associée à une utilisation d'une première de la ou des séquences pour un premier des contextes; générer une règle indiquant si la première séquence doit être incluse ou exclue pour une procédure d'acquisition d'image ultérieure ayant le premier contexte sur la base de la ou des métriques; recevoir un ordre d'image ultérieur correspondant à la procédure d'acquisition d'image ultérieure, l'ordre d'image ultérieur ayant un contexte ultérieur; déterminer si le contexte suivant correspond au premier contexte; et lorsque le contexte ultérieur correspond au premier contexte, déterminer une indication indiquant si la première séquence doit être incluse ou exclue dans la procédure d'acquisition d'image ultérieure sur la base de la règle; dans lequel la au moins une métrique comprend un pourcentage de captures d'images dans les procédures d'acquisition précédentes pour le premier contexte dans lequel la première séquence est utilisée, un pourcentage d'interprétations dans les procédures d'acquisition précédentes pour le premier contexte dans lequel les captures d'images sont utilisées, ou une combinaison de ceux-ci; le procédé comprenant en outre la mise en oeuvre automatique de l'indication pour la première séquence dans la procédure d'acquisition d'image suivante.

2. Procédé selon la revendication 1, dans lequel les données comprennent au moins un dossier médical électronique (EMR), des informations de commande d'images englobant une raison pour une commande d'images, des informations d'utilisation de séquence, ou une combinaison de celles-ci.

3. Procédé selon la revendication 1, dans lequel la règle identifie une priorité de la première séquence pour le premier contexte, dans lequel la priorité est de savoir si la première séquence est obligatoire pour le premier contexte, suggérée pour le premier contexte, ou doit être omise pour le premier contexte.

4. Procédé selon la revendication 1, dans lequel le premier contexte est basé sur un premier protocole qui comprend la première séquence, une première commande d'images correspondant à une première procédure d'acquisition d'images, un premier motif de la première commande d'images, un profil clinique d'au moins un patient ayant subi la première procédure d'acquisition d'images, ou une combinaison des deux.

5. Procédé selon la revendication 1, comprenant en outre: recevoir une entrée manuelle pour une autre règle, l'autre règle étant une règle modifiée par l'utilisateur, une règle générée par l'utilisateur, une règle de sécurité, une règle de conformité ou une combinaison de celles-ci.

6. Procédé selon la revendication 1, dans lequel les procédures d'acquisition d'images correspondent à des opérations de capture d'images réalisées avec une procédure de radiographie, une procédure de tomographie assistée par ordinateur (CT), une procédure d'imagerie par résonance magnétique (MRI), une procédure d'échographie, une procédure de tomographie par émission de positrons (PET), une procédure de tomographie par émission monophotonique (SPECT), ou une combinaison de ces procédures.

7. Serveur de flux de travail, comprenant: un émetteurrécepteur communiquant via un réseau de communications, l'émetteur-récepteur configuré pour recevoir des données associées à des procédures d'acquisition d'images précédentes qui ont été effectuées, les procédures d'acquisition d'images ayant des ordres d'images respectifs, les ordres d'images ayant des contextes respectifs, chacune des procédures d'acquisition d'images ayant été utilisée au moins une séquence définissant des paramètres pour capturer une image; une mémoire stockant un programme exécutable; et un processeur qui exécute le programme exécutable qui amène le processeur à effectuer des opérations, comprenant: déterminer au moins une métrique associée à une utilisation d'une première de la ou des séquences pour un premier des contextes; générer une règle indiquant si la première séquence doit être sélectionnée pour une procédure d'acquisition d'image ultérieure ayant le premier contexte sur la base de la ou des métriques; recevoir un ordre d'image ultérieur correspondant à la procédure d'acquisition d'image ultérieure, l'ordre d'image ultérieur ayant un contexte ultérieur; déterminer si le contexte suivant correspond au premier contexte; et lorsque le contexte ultérieur correspond au premier contexte, déterminer une indication indiquant si la première séquence doit être incluse ou exclue dans la procédure d'acquisition d'image ultérieure sur la base de la règle; et mettre en oeuvre automatiquement l'indication pour la première séquence dans la procédure d'acquisition d'image ultérieure; dans lequel la au moins une métrique comprend un pourcentage de captures d'images dans les procédures d'acquisition précédentes pour le premier contexte dans lequel la première séquence est utilisée, un pourcentage d'interprétations dans les procédures d'acquisition précédentes pour le premier contexte dans lequel les captures d'images sont utilisées, ou une combinaison de ceux-ci.

8. Serveur de flux de travail selon la revendication 8, dans lequel la règle identifie une priorité de la première séquence pour le premier contexte, dans lequel la priorité est de savoir si la première séquence est obligatoire pour le premier contexte, suggérée pour le premier contexte, ou doit être omise pour le premier contexte.

9. Serveur de flux de travail selon la revendication 8, dans lequel le premier contexte est basé sur un premier protocole qui comprend la première séquence, une première commande d'images correspondant à une première procédure d'acquisition d'images, une première raison de la première commande d'images, un profil clinique de au moins un patient ayant subi la première procédure d'acquisition d'images, ou une combinaison de celles-ci.

10. Serveur de flux de travail selon la revendication 8, dans lequel l'émetteur-récepteur reçoit en outre une entrée manuelle pour une autre règle, dans lequel l'autre règle est l'une parmi une règle modifiée par l'utilisateur, une règle générée par l'utilisateur, une règle de sécurité, une règle de conformité, ou une combinaison de ceux-ci.
